# EUROPÄISCHE PATENTSCHRIFT

(11) **EP 1 194 442 B1**
(45) Veröffentlichungstag und Bekanntmachung des Hinweises auf die Patenterteilung: **24.08.2005**
(21) Anmeldenummer: 00938817.4
(22) Anmeldetag: 23.06.2000
(51) Int. Cl.: C07K 1/20, C07K 14/765, C07K 16/06, C07K 14/81, C07K 14/79

(54) **VERFAHREN ZUR CHROMATOGRAPHISCHEN FRAKTIONIERUNG VON PLASMA ODER SERUM, SO ERHALTENE PRÄPARATE UND DEREN VERWENDUNG**
METHOD FOR CARRYING OUT THE CHROMATOGRAPHIC FRACTIONATION OF PLASMA OR SERUM, PREPARATIONS OBTAINED THEREBY AND THEIR USE
PROCEDE DE FRACTIONNEMENT PAR CHROMATOGRAPHIE DE PLASMA OU DE SERUM, PREPARATIONS AINSI OBTENUES ET LEUR UTILISATION

(30) Priorität: 14.07.1999 DE 19932782
(43) Veröffentlichungstag der Anmeldung: 10.04.2002
(73) Patentinhaber: BIOTEST PHARMA GMBH, D-63303 Dreieich (DE)
(72) Erfinder: KOTHE, Norbert, D-61476 Kronberg/Taunus (DE); RUDNICK, Dieter, D-63303 Dreieich (DE); KLOFT, Michael, D-64291 Darmstadt (DE)
(74) Vertreter: Alber, Norbert
(86) Internationale Anmeldenummer: PCT/EP2000/005827
(87) Internationale Veröffentlichungsnummer: WO 2001/005809

(56) Entgegenhaltungen:
- EP-A- 0 339 919
- EP-A- 0 717 049
- US-A- 5 429 746
- HRKAL Z ET AL: "HYDROPHOBIC INTERACTION CHROMATOGRAPHY OF SERUM PROTEINS ON PHENYL SEPHAROSE CL-4B" JOURNAL OF CHROMATOGRAPHY, Bd. 242, Nr. 2, 1982, Seiten 385-388, XP000946223 ISSN: 0021-9673
- GOHEEN S C ET AL: "PURIFICATION OF HUMAN SERUM GAMMA GLOBULINS BY HYDROPHOBIC INTERACTION HIGH-PERFORMANCE LIQUID CHROMATOGRAPHY" JOURNAL OF CHROMATOGRAPHY, Bd. 326, 1985, Seiten 235-241, XP000946239 ISSN: 0021-9673 in der Anmeldung erwähnt
- SZEPESZ L ET AL: "HIGH PERFORMANCE HYDROPHOBIC INTERACTION CHROMATOGRAPHY OF PROTEINS" LC-GC INTERNATIONAL (LIQUID AND GAS CHROMOTOGRAPHY),US,EUGENE, OR, Bd. 5, Nr. 11, 1992, Seiten 24-29, XP000350924

## Beschreibung

Die Erfindung betrifft das in den Ansprüchen beschriebene Rezyklisierungs-Verfahren zur chromatographischen Fraktionierung von Plasma oder Serum, insbesondere Humanplasma/ -Serum an einer hydrophoben Interaktionsphase mittels eines stufenweisen Salzgradienten.

Erste Versuche zur industriellen Fraktionierung von Humanplasma wurden schon in den 40iger Jahren durchgeführt. Die hierbei angewandte Methode basiert auf der Fällung von Proteinfraktionen mittels Alkohol, wobei durch Variation von pH, Ionenstärke, Temperatur und Alkoholkonzentration in diesen Fraktionen verschiedene Plasmaproteine in angereicherter Form erhalten werden.

Von besonderer Bedeutung für die therapeutische Anwendung sind die nach diesem Prozess gewonnenen Fraktionen, die Immunglobuline und Albumin enthalten. Eine Übersicht über o. g. Methoden ist den Übersichtsartikeln (J.E. More, M.J. Harvey, Blood Separation and Plasma Fractionation, Wiley-Liss, New York 1991, 261-306 und P. Kistler, H. Friedli, Methods of Plasma Protein Fractionation, Academic Press, London, 1980, 3-15) zu entnehmen.

Neben Alkohol werden auch andere Fäl!reagenzien, wie Ammoniumsulfat und Polyethylenglycol, zur Gewinnung von Plasmafraktionen eingesetzt (A.P. Phillips, K.L. Martin, W.H. Horton, The choice of methods for immunoglobulin IgG purification: Yield and Purity of Antibody Activity, J. of Immunological Methods, 74 (1984) 385-393, A. Polson et al., The Fractionation of Protein Mixtures by Linear Polymers of High Molecular Weight, Biochem.

Biophys. Acta, 82 (1964) 463-475; P.D. Gorevic et. al., Methods in Enzymology, Vol. 116, 1985, 3-25; DE 2936047C2).

Prinzipiell weisen alle Fällmethoden zur Isolierung von Plasmaproteinen einige Nachteile auf. Die zur Fällung eingesetzten Agentien bewirken eine partielle Denaturierung der separierten Proteine. Dies ist an der Bildung von Aggregaten und damit verbundenen Unverträglichkeiten bei der therapeutischen Anwendung zu erkennen. Um aus den so gewonnenen Proteinfraktionen verträgliche Präparate zu erhalten, sind weitere aufwendige Reinigungsschritte norivendig.

Ein weiterer Nachteil der Methoden besteht darin, dass Fällungsreaktionen, speziell bei so komplexen Gemischen wie Humanplasma, niemals quantitativ verlaufen. Dies und die zusätzlich notwendigen Reinigungsschritte führen zu beträchtlichen Ausbeuteverlusten.

Diese Verfahren stellen somit keine optimale Nutzung des wertvollen Ausgangsmaterials, insbesondere von Humanplasma oder Humanserum dar.

Aus diesem Grunde wurden Versuche unternommen, die Plasmafraktionierung mit schonenderen Methoden und höheren Ausbeuten durchzuführen. Hierzu bieten sich adsorptive Methoden an.

Spezielle Eigenschaften der Proteine, wie Ladung, Hydrophobizität, Größe und charakteristische Bindungseigenschaften werden dazu genutzt, um eine Bindung der Proteine an geeignete Liganden zu bewirken.
Besonders vorteilhaft ist es, wenn diese Liganden an einen wasserunlöslichen stationären Träger gebunden sind. Die Bindung der Proteine an den substituierten Träger kann im Batch- oder als säulenchromatographisches Verfahren durchgeführt werden, wobei sich die chromatographische Methode als besonders vorteilhaft erweist. Zur chromatographischen Isolierung von Plasmaproteinen wurden Anionen- und Kationenaustauscher, Affinitäts- und hydrophobe Phasen sowie ausschlußchromatographische Materialien eingesetzt.

Diese chromatographischen Methoden wurden bisher in erster Linie zur Feinreinigung von gefällten Plasmafraktionen verwendet.

Im Folgenden sind einige Beispiele aufgelistet.

In der EP 0447585B1 ist die Isolierung von Immunglobulin G aus Cohn Paste II beschrieben.

EP 0352500B1 beinhaltet die Herstellung von Immunglobulin M aus mit Alkohol präzipitierter Paste III.

Transferrin (DE 3733181C1), α₁-Antitrypsin (EP 0717049A1, US 5,610,285) und Antithrombin III (EP 0844254A2) können mit Hilfe unterschiedlicher chromatographischer Methoden aus Cohn-Paste IV isoliert werden.

Cohn-Paste V dient als Ausgangsmaterial für die chromatographische Albumin-Herstellung (EP 0792887A1) sowie zur Aufreinigung von α₁-saurem Glycoprotein (US 5,739,293).

Ebenso können alkoholhaltige Überstände der Cohn-Fraktionierung als Ausgangsmaterial für eine chromatographische Albumin-Herstellung eingesetzt werden (EP 0402205B1).

Eine Kombination von Alkoholfällung und Chromatographie zur Gewinnung von Plasmaproteinen ist in US 5,138,034 beschrieben.

Auch die direkte Gewinnung von Immunglobulin G und Albumin aus Humanplasma, ohne vorherige Fällung und Abtrennung eines Präzipitates, mittels lonenaustauschchromatographie ist in DE 3640513C2 und WO 94/29334 beschrieben.

Eine Übersicht über die Anwendung chromatographischer Methoden zur Gewinnung von Proteinen aus Humanplasma findet sich in: J.M. Curling, Separation of Plasma Proteins, Pharmacia Fine Chemicals AB, Uppsala, Sweden, 1993.

Wie schon oben erwähnt, weisen alle beschriebenen Verfahren entscheidende Nachteile auf. So führen alle über Fällreaktionen gewonnenen Plasmaproteine zu erheblichen Ausbeuteverlusten und erfordern aufwendige zusätzliche Reinigungsschritte, um die therapeutische Anwendung zu ermöglichen. Außerdem ist ein großer technischer Aufwand zur Kühlung der Reaktionsgefäße, Zentrifugen oder Filter notwendig.

Die mittels lonenaustauschchromatographie aus Plasma gewonnenen Produkte erfordern eine aufwendige Vorkonfektionierung des Humanplasmas.

Zur Durchführung der Ionenaustauschchromatographie muß das Plasma auf eine definierte, niedrige lonenstärke eingestellt werden. Dies kann durch Verdünnen oder durch Umpufferung erreicht werden.

Hierbei entstehen große Volumina, die im technischen Maßstab die Menge des zu prozessierenden Plasmas limitieren. Darüber hinaus muß durch einen zusätzlichen Schritt vor der Chromatographie das im Humanplasma enthaltene Fibrinogen entfernt werden, um ein Verblocken der Säule zu verhindern.

S. Coheen et al. beschreiben in J. of Chromatography, 326 (1985), 235-241 ein Verfahren zur Fraktionierung von Humanserum. Dabei wird eine Bio-Gel TSK Phenyl-5PW-Säule mit der Ausgangslösung bestückt und anschließend bei 0°C mittels eines linearen Ammoniumsulfatsatzgradienten in 0,1 M Natriumphosphatpuffer eluiert. Eine solche lineare Eluierung kann nur im analytischen Maßstab vorgenommen werden, da ansonsten bei Auftragen der Ausgangslösung und Anwendung der genannten Anfangskonzentration von 1,7 M die Säule verstopfen würde, wenn man im präparativen Maßstab arbeiten würde. Darüberhinaus muß bei 0°C gearbeitet werden, um die Auflösung des Chromatogramms zu verbessern. Eine solche Verfahrensweise ist auf einen Prozess im präparativen Umfang wegen des technischen Aufwands nicht übertragbar.

Aus Goheen, Journal Chromatography, 326, 1985, Seiten 235 bis 241, ist ein Verfahren zur Fraktionierung von Humanserum ohne Rivanolfällung mittels eines linearen Ammoniumsulfatsalzgradienten durch eine hydrophobe Interaktionschromatographie (HIC) bekannt.

Der Erfindung liegt die Aufgabe zugrunde, ein im technischen-Maßstab wirtschaftlich durchführbares Rezyklisierungs-Verfahren zur präparativen Fraktionierung von Plasma, insbesondere Humanplasma, oder Serum, insbesondere Humanserum, bereitzustellen, das in hohen Ausbeuten native, unmodifizierte Plasmaproteine liefert. Hierbei sollte ein Präzipitieren und Auflösen von therapeutisch relevanten Proteinen überwiegend vermieden werden und der Prozess bei Raumtemperatur ablaufen.

Erfindungsgemäß wird diese Aufgabe durch die Merkmale des Anspruchs 1 gelöst. Bevorzugte Weiterbildungen sind den abhangigen Anspruchen zu entnehmen.

Gemäß der Erfindung wird ein Rezyklisierungsverfahren zur präparativen Fraktionierung von Plasma oder Serum durch hydrophobe Interaktionschromatographie unter Verwendung eines stufenweisen Salzgradienten, wobei wenigstens eine Immunglobulin und eine Albumin enthaltende Fraktion erhalten wird, bereitgestellt, bei dem
(a) eine Serum oder Plasma enthaltende Ausgangslösung in Gegenwart einer hohen Salzkonzentration auf die hydrophobe Chromatographiephase aufgetragen,
(b) eine erste Albumin enthaltende Fraktion kontinuierlich gesammelt,
(c) die aus (b) erhaltene erste Fraktion kontinuierlich ankonzentriert,
(d) das aus (c) erhaltende Permeat mit hoher Salzkonzentration kontinuierlich dem Vorratsgefäß der Pufferlösung 1 rückgeführt, dann
(e) mit einem Mischpuffer mit niedriger Salzkonzentration, hergestellt aus der rezyklierten Pufferlösung 1 mit hoher Salzkonzentration aus (d) und einer salzfreien Pufferlösung 2, oder der Pufferlösung 2 alleine eluiert, und
(f) das Eluat als eine zweite Immunglobulin enthaltende Fraktion gesammelt
werden.

Erfindungsgemäß wird Plasma oder Serum, insbesondere humanen Ursprungs, bevorzugt ein von Gerinnungsfaktor VIII und/oder den Gerinnungsfaktoren des PPSB-Komplexes befreites Plasma oder Serum, insbesondere humanen Ursprungs an einer hydrophoben Phase unter Anwendung eines stufenweisen Salzgradienten chromatographiert. Als Salz eignet sich insbesondere Ammoniumsulfat.

Überraschenderweise wurde gefunden, dass mit Hilfe der hydrophoben Interaktionschromatographie Plasma oder Serum in wenigstens eine Immunglobulin- und eine Albuminhaltige Fraktion im präparativen Maßstab getrennt werden kann, wobei die Chromatographie bei Raumtemperatur, ohne aufwendige Kühlvorrichtungen, durchgeführt werden kann.

Im folgenden wird das erfindungsgemäße Verfahren näher erläutert.

### 1. Fraktionierung

Erfindungsgemäß kann (Human-)Plasma oder (Human-)Serum verwendet werden, welches von Gerinnungsfaktoren befreit sein kann. Darüberhinaus kann auch tierisches Plasma oder Serum mit oder insbesondere ohne Gerinnungsfaktoren eingesetzt werden. Das Ausgangsmaterial kann sowohl von normalen Spenderpools als auch von ausgewählten (selektierten) Spendern mit hohen Antikörpertitern gegen virale, bakterielle oder zelluläre Antigene gewonnen werden. Bei selektiertem Ausgangsmaterial (Hyperimmunglobulin) wird bevorzugt solches mit hohen Titern gegen CMV, Hepathitis B, Varicella, Tetanus oder Anti-D gewählt.

Vorzugsweise wird als Ausgangsmaterial für das erfindungsgemäße Verfahren ein von den o.g. Gerinnungsfaktoren befreites Plasma, insbesondere Humanplasma oder -serum eingesetzt. Die Abtrennung der wertvollen Gerinnungsfaktoren aus Plasma, die selbst von großem therapeutischen Nutzen sind, ist bekannt.

So wird beispielsweise das aus einem Auftauprozess gewonnene Kryopräzipitat als Ausgangsmaterial für die Herstellung von Faktor VIII-Konzentrat verwendet und die Faktoren II, VII, IX und X (PPSB-Komplex) werden durch Adsorption an einen Anionenaustauscher isoliert und in gereinigter Form ebenfalls therapeutisch eingesetzt.

Zur Gewinnung des bevorzugten Ausgangsmaterials für das erfindungsgemäße Verfahren kann daher insbesondere ein nach den Richtlinien des Blutspendewesens mittels Plasmapherese gewonnenes Plasma bei + 4°C aufgetaut und das Kryopräzipitat durch Zentrifugation entfernt werden. Hieraus kann Faktor VIII hergestellt werden.
Die Gerinnungsfaktoren des PPSB-Komplexes werden durch Adsorption an einen Anionenaustauscher, z. B. einen mit Diethylaminoethyl-Gruppen substituierten vernetzten Dextran (wie z.B. DEAE-Sephadex® A50) abgetrennt.

Dem Ausgangsmaterial, mit oder ohne Gerinnungsfaktoren, wird vorzugsweise pro Liter soviel Gradientensalz, im Falle von Ammoniumsulfat soviel festes Ammoniumsulfat zugefügt, daß nach mehreren Stunden, z.B. 3-20, Rühren bei Raumtemperatur, ggf. nach Zugabe von Wasser für Injektionszwecke die Leitfähigkeit einer entsprechenden Salz- insbesondere Ammoniumsulfat-Lösung, vorliegt, die für den Chromatographiebeginn, entsprechend der gewählten Anfangskonzentration an Salz, insbesondere Ammoniumsulfat, vorgesehen ist. Nach Zugabe von 0,5-5 % Filterhilfsmittel, z.B. Standard Super Cell oder anderer geeigneter Filterhilfsmittel wie z.B. Perlite, Harbolite oder Celite, wird die Lösung filtriert.
Das Filtrat wird als Ausgangslösung für die Chromatographie eingesetzt.
Die hydrophobe Interaktionsphase wird dabei vorzugsweise auf die gewünschte Ausgangskonzentration an Salz. insbesondere Ammoniumsulfat entsprechend der gewählten Konzentration in der Ausgangslösung äquilibriert.

Die chromatographische Trennung beruht auf der Wechselwirkung von hydrophoben Domänen der Proteinmoleküle mit hydrophoben Gruppen auf der stationären chromatographischen Phase. Unter physiologischen Bedingungen sind die hydrophoben Gruppen der Proteinmoleküle nicht frei zugänglich, so dass eine Bindung an eine hydrophobe Interaktionsphase nicht stattfindet. Durch Hinzufügen des Salzes, insbesondere von Ammoniumsulfat wird die Hydrathülle des Proteins verringert, damit stehen die hydrophoben Domänen für eine hydrophobe Interaktion zur Verfügung.

Die Hydrophobizität der stationären Phase wird erfindungsgemäß somit so gewählt, dass eine Wechselwirkung bestimmter Proteine mit der Phase stattfinden kann. Die Bindung an die Phase hängt einerseits von der Hydrophobizität der chromatographischen Matrix, andererseits von der Salz- insbesondere der Ammoniumsulfatkonzentration der Lösung ab. Da bei hohen Salz- insbesondere Ammoniumsulfatkonzentrationen eine Präzipitation der Proteine erfolgt, wird die Salz-z.B. die Ammoniumsulfatkonzentration entsprechend der Hydrophobizität der chromatographischen Phase so gewählt, dass die gewünschten Wechselwirkungen bei einer Salz- bzw. Ammoniumsulfatkonzentration, die überwiegend nicht zur Präzipitation führt, stattfinden.

Dies ergibt sich beispielsweise aus Tabelle 1, worin in Abhängigkeit von der Ammoniumsulfatkonzentration der Anteil an noch in Lösung befindlichen Proteinen angegeben ist. Bei Werten von 1,4 Mol/l Ammoniumsulfat und darüber wird kein befriedigendes Ergebnis erhalten.

Somit zeigt sich, daß die Konzentration an Salz, insbesondere Ammoniumsulfat < 1,4 Mol/l sein sollte.

Zur wirksamen Trennung von Immunglobulin- und Albuminfraktion wird dementsprechend vorzugsweise bei einer Ammoniumsulfatkonzentration von < 1,4 Mol/l, auf die gewünschtenfalls die Chromatographie-Phase und die Ausgangslösung eingestellt wird, begonnen, vorzugsweise mit einem Puffer dieser Salzkonzentration nachgewaschen und nachfolgend die Ammoniumsulfatkonzentration gesenkt.

Besonders bevorzugt wird die Trennung vorgenommen, wenn die hohe Konzentration 0,6 bis < 1,4 Mol/l, vorzugsweise bis 1,3 Mol/l, insbesondere 0,6 bis 1,2 Mol/l, ganz besonders 0,7 bis 1 Mol/l, insbesondere 0,8 bis 0,9 Mol/l, und die abgesenkte Konzentration 0,4 bis 0 Mol/l, insbesondere 0,3 bis 0 Mol/l beträgt.

Bei einem solchen Konzentrationsgefälle wird zunächst eine Albumin-haltige Fraktion und dann eine Immunglobulin-haltige Fraktion erhalten. Letztere kann noch Lipide enthalten, die vorteilhafterweise entfernt werden. Hierzu kann im zweiten Schritt eine weitere Konzentrationsstufe des Ammoniumsulfats eingesetzt werden, z.B. durch Beginn bei 0,4, insbesondere 0,3 Mol/l, bis 0,1 Mol/l , insbesondere 0,3 bis 0,15 Mol/l, ganz besonders bei 0,3 Mol/l und anschließende Absenkung auf kleiner 0,1 Mol/l bis 0 Mol/l. Dabei werden die Lipide erst bei der niedrigsten, ggf. Null (0 Mol/l)-Konzentration von der Phase entfernt und damit aus der Immunglobulinfraktion, die die Phase bei höheren Ammoniumsulfatkonzentrationen verläßt, abgetrennt.

Bei der Chromatographie werden die üblichen Bedingungen, die für solche Phasen bekannt sind, eingehalten, wie z.B. pH-Wert (z.B. 7,0), Natrium(hydrogen)Phosphatpuffer oder Trishydroxymethylaminomethan als Elutionsmittel. Phosphatpuffer sind besonders bevorzugt (z.B. 0,01 M NaH₂PO₄).

Als hydrophobe lnteraktionsphasen, sind für den erfindungsgemäßen Prozess die bekannten Materialien geeignet. Hierzu gehören z.B. Phenyl- oder Alkyl-substituierte Phasen auf der Basis von Copolymeren aus Glycidyl-methacrylat und Ethylenglycol-dimethacrylat, Copolymere aus Polystyrol und Divinylbenzol oder mit Dextran oder Polymeren gecoatete Kieselgele.

Als besonders geeignet erwiesen sich Alkyl- und ganz besonders Phenyl-substituierte Copolymere aus Glycidyl-methacrylat und Ethylenglycoldimethacrylat.

Diese sind unter den Handelsnamen TSK-Phenyl 5PW® und Toyopearl-Phenyl 650® kommerziell erhältlich.
Hierfür kann erfindungsgemäß beispielsweise eine Anfangs-Ammoniumsulfatkonzentration von 0,7 bis 1,0 Mol/l, vorzugsweise von 0,8 bis 1,0, insbesondere 0,9 Mol/l, für die chromatographische Separation gewählt werden.
Wie aus Tabelle 2 ersichtlich ist, führt die chromatographische Trennung an TSK-Phenyl 5PW® bei solchen Konzentrationen zu besonders guten Ergebnissen.

So kann, wie oben beschrieben, die auf eine Ammoniumsulfatkonzentration von 0,9 Mol/l eingestellte Ausgangslösung auf die äquilibrierte hydrophobe Chromatographiephase aufgetragen werden.Bei dieser Salzkonzentration erhält man eine Fraktion 1, die die Phase ungebunden passiert. Die gebundenen Proteine werden mit 0,01 Mol/l Natriumphosphat, pH 7,0 als Fraktion 2 von der Phase abgelöst, d.h. die Ammoniumsulfatkonzentration wird hier auf 0 Mol/l abgesenkt.

In **Tabelle 3** ist die Zusammensetzung der erhaltenen chromatographischen Fraktionen dargestellt.
Wie hieraus ersichtlich ist, erhält man eine Fraktion 1, die als Albuminfraktion bezeichnet wird und die in der Zusammensetzung einem Überstand II/III der Alkoholfraktionierung nach Cohn sehr ähnlich ist. Sie enthält die wichtigen Proteine Albumin, Transferrin, Antithrombin III und α-Antitrypsin.
In der Fraktion 2 sind fast quantitativ alle lmmunglobuline enthalten und ähnelt daher der nach dem Kälte-Äthanol-Verfahren erhaltenen Zusammensetzung einer Paste II/III.

Wie aus Tabelle 3 weiter zu ersehen, sind in der Fraktion 2 auch alle Lipide und Lipoproteine enthalten. Diese können die Weiterverarbeitung der Fraktion 2 zu therapeutisch anwendbaren Proteinlösungen erschweren, so dass es sich als vorteilhaft erwiesen hat, einen weiteren Elutionsschritt zur Gewinnung einer 3. Fraktion in den Prozess zu integrieren.
Hierzu kann nach Gewinnung der Fraktion 1 bei der höchsten Konzentration, z.B. 0,9 Mol/l Ammoniumsulfat begonnen und dann mit einem Stufengradienten weitergearbeitet werden. Beispielsweise kann bei 0,3 Mol/l Ammoniumsulfat die Fraktion 2 isoliert werden.

Diese immunglobulinhaltige Fraktion 2 unterscheidet sich in der Proteinzusammensetzung nicht von der oben beschriebenen Fraktion (Tabelle 3, Fraktion 2), außer, dass der Gehalt an Lipoproteinen kleiner 5 % ist.

Die Lipoproteinfraktion bleibt bei 0,3 Mol/l und höheren Ammoniumsulfatkonzentrationen an die Chromatographiephase gebunden und kann mit 0,01 Mol/l Natriumphosphat, pH 7,0 als Fraktion 3 von der Phase eluiert werden, d.h. hier wird die Ammoniumsulfatkonzentraion auf 0 Mol/l abgesenkt.

Vorzugsweise werden somit 3 Fraktionen bei der erfindungsgemäßen hydrophoben Interaktionschromatographie separiert, nämlich zunächst die Albuminfraktion, dann die Immunglobulinfraktion und dann die Lipoproteinfraktion.

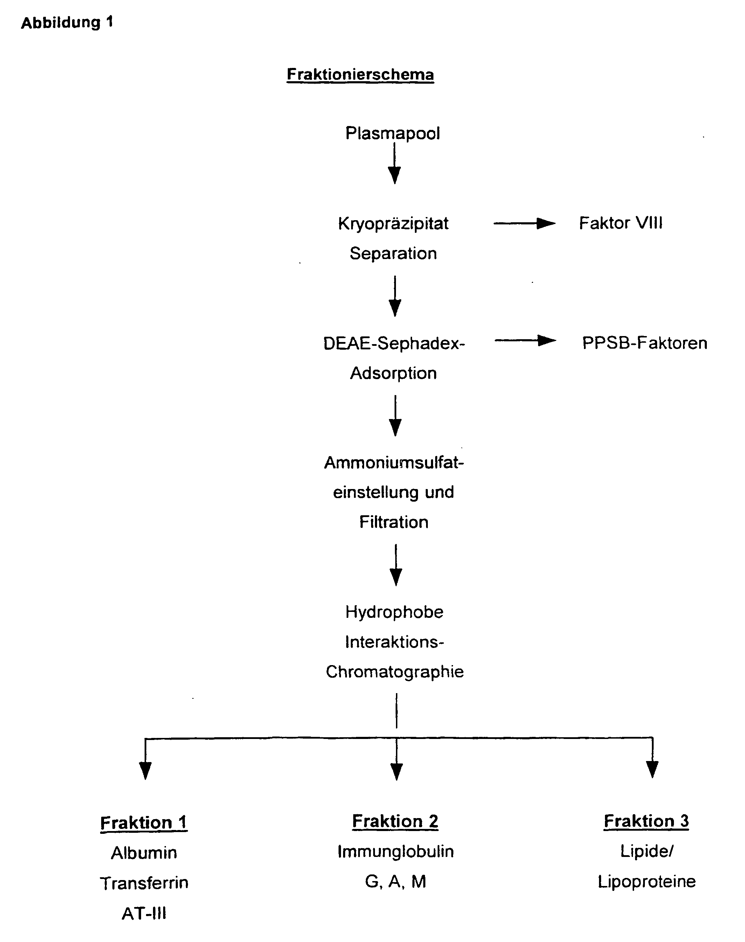

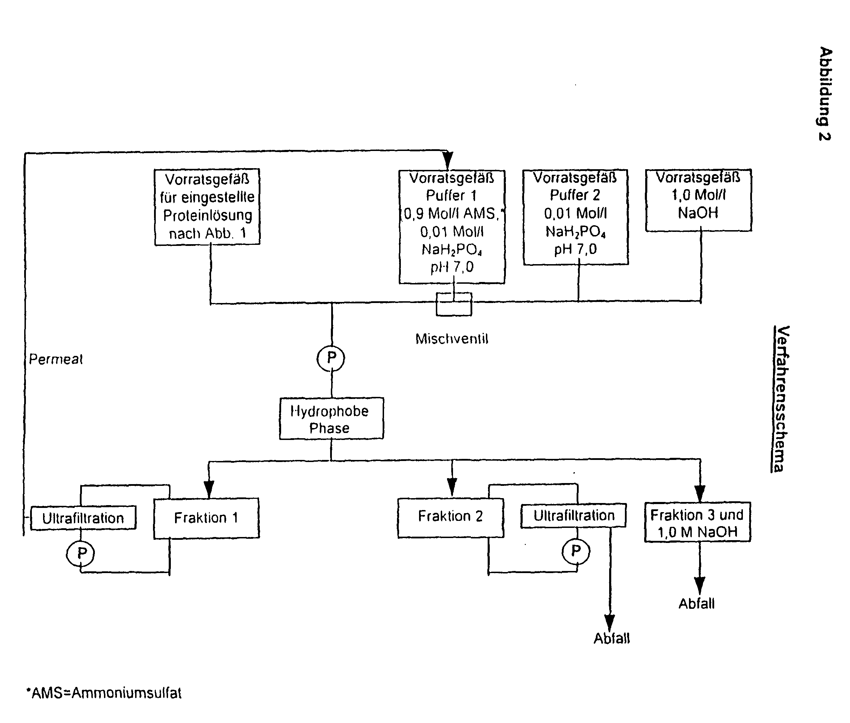

Schematisch ist der erfindungsgemäße Prozess in **Abbildung 1** dargestellt.

### 2. Rezyklisierung

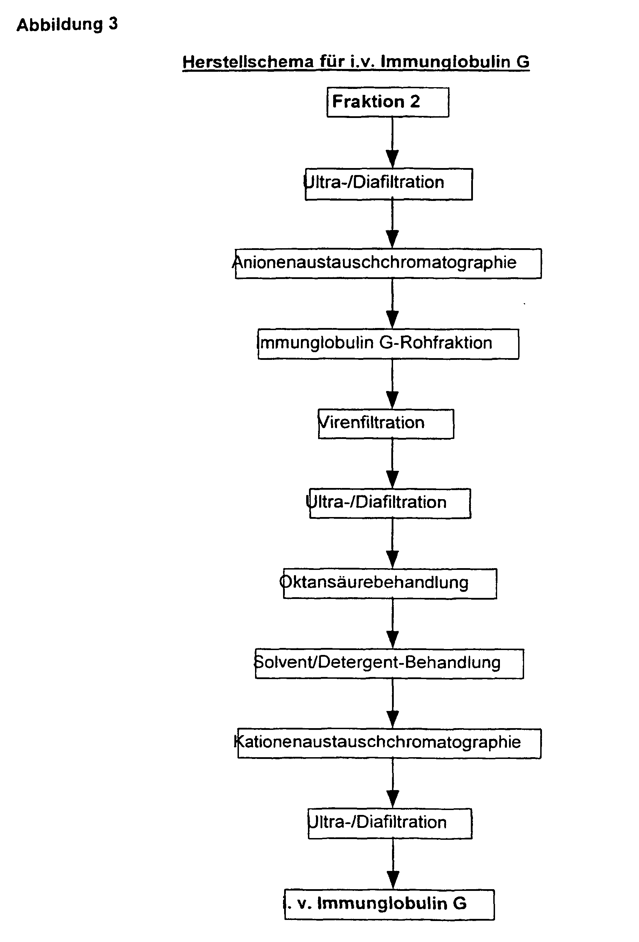

Bei der großtechnischen Anwendung des Verfahrens werden Puffersalz- wie Ammoniumsulfat-haltige Pufferlösungen in beträchtlichen Mengen benötigt. Um diesen Verbrauch möglichst zu minimieren, wird erfindungsgemäß ein Rezyklisierungsverfahren angewendet, wie in Abbildung 2 dargestellt.

Dabei kann der auf die gewünschte Konzentration eingestellte z.B. Ammoniumsulfatpuffer 1 (hohe Konzentration) als Permeat aus der gewonnenen ersten Fraktion z.B. mittels geeigneter Ultrafiltrationsmembranen rückgeführt werden, während die erste Fraktion kontinuierlich gesammelt und ankonzentriert wird. Zwecks Abtrennung der zweiten und ggf. dritten Fraktion wird dann ein Puffer mit der jeweils gewünschten Konzentration durch Mischen des Ammoniumsulfatpuffers 1 und einem Nicht-Gradientensalz, d.h. Nicht-Ammoniumsulfatpuffer 2 oder durch alleinigem Einsatz des Puffers 2 abgesenkt wie erfindungsgemäß angegeben und die zweite bzw. dritte Fraktion abgetrennt, je nachdem wie viele Fraktionen gewünscht sind.

Vorzugsweise werden, wie oben beschrieben, drei Fraktionen hergestellt durch Anwendung eines Stufengradienten nach Abtrennung der Fraktion 1, wobei die vorstehend angegebenen Ammoniumsulfatkonzentrationen gewählt werden.

Das kontinuierliche Ankonzentrieren der Fraktionen kann z.B. mittels Ultrafiltration erfolgen.

Es wird femer bevorzugt, die Chromatographiephase nach je einem Zyklus mit Natronlauge aus einem Vorratsbehälter 3 zu reinigen, wobei gleichzeitig eine Sterilisation erfolgt.

Beispielsweise kann nach der Beladung der Säule mit der auf einem Puffer 1 eingestellten und filtrierten Plasmaprotein-Lösung zur Gewinnung der Fraktion 1 mit Puffer 1 (z.B. 0,9 Mol/l Ammoniumsulfat, 0,01 Mol/l NaH₂PO₄ pH 7,0) nachgewaschen werden, um die gesamte Fraktion 1 zu gewinnen.Die so gewonnene Fraktion 1 wird aufgefangen und kontinuierlich über eine Ultrafiltrationseinheit mit einem cut off von 10 KD ankonzentriert. Das hierbei gewonnene Permeat wird in den Vorratskessel für Puffer 1 zurückgeführt.

Zur Elution der Fraktion 2 wird die Salz- bzw. Ammoniumsulfatkonzentration wie angegeben abgesenkt, wobei beispielsweise ein Mischpuffer hergestellt werden kann oder ein Puffer 2, welcher kein Salz, z.B. Ammoniumsulfat enthält, allein gewählt wird, je nachdem, wie viele Trennstufen gewünscht sind.

Bei einem Stufengradient können, beispielsweise zur Elution der Fraktion 2, die Puffer 1 (0,9 Mol/l Ammoniumsulfat, 0,01 Mol/l NaH₂PO₄, pH 7,0) und Puffer 2 (0,01 Mol/l NaH₂PO₄, pH 7,0) online im Verhältnis z.B. 1:3 gemischt werden. Dieses Mischungsverhältnis ergibt einen Elutionspuffer für die Fraktion 2 mit der gemischten Konzentration z.B. von 0,3 Mol/l Ammoniumsulfat, 0,01Mol/l NaH₂PO₄, pH 7,0.
Die gesammelte Fraktion 2 wird ebenfalls online über eine Ultrafiltrationseinheit mit einem cut off von 10-30 KD ankonzentriert. Das hierbei anfallende Permeat wird verworfen.

Mit Puffer 2 (0,01 Mol/l NaH₂PO₄, pH 7,0) werden sodann die Lipoproteine von der hydrophoben Phase losgelöst und verworfen.

Dieses Verfahren kann unter Änderungen der Puffer/Salz-konzentrationen und Mischverhältnisse im erfindungsgemäßen jeweils gewünschten Bereich und üblichen, dem Fachmann für die genannten Chromatographiephasen bekannten Bedingungen variiert werden. So kann z.B. bei einer Puffer 1-Konzentration von 1 Mol/l mit Puffer 2 eine 1:1 - 1:5 etc. Mischung hergestellt werden.

Beim Prozessieren von großen Mengen werden mehrere chromatographische Zyklen durchgeführt.
Vorzugsweise wird daher nach jedem Separationszyklus die hydrophobe Phase mit 1,0 Mol/l NaOH gereinigt und gleichzeitig sterilisiert. Die Natronlauge befindet sich im Vorratsbehälter 3.

### 3. Weiterverarbeitung

Die nach dem erfindungsgemäßen Verfahren an der hydrophoben Phase gewonnenen Fraktionen 1 und 2 können nach bekannten Verfahren, wie z.B. denen in den eingangs erwähnten Druckschriften beschriebenen zu therapeutisch anwendbaren hochreinen Plasmaprotein-Lösungen weiterverarbeitet werden. Solche Präparate werden bei jeweiligen Mangel- oder Krankheitszuständen eingesetzt, z.B. können bei Infektionen Präparate, die aus selektiertem Ausgangsmaterial erfindungsgemäß hergestellt wurden, wie z.B. solche enthaltend anti-CMV oder anti-HBs, anti-Varicella, Tetanus oder Anti-D bei entsprechender Infektion verabreicht werden.

Mengen und Abreichungsform sind hierbei jeweils bekannt, z.B. als Injektionen oder i.m.-Injektion oder i.v.-Infusion, wobei die pharmazeutische Zusammensetzung neben dem Wirkstoff die bekannten Hilfsstoffe aufweisen können. Hierzu gehören z.B. Elektrolyte, Aminosäuren oder Zucker.

### a) Immunglobulin G

Beispielsweise kann aus der Immunglobulinfraktion (Fraktion 2) ein i. v. verträgliches, insbesondere Immunglobulin G hergestellt werden. Hierzu können bekannte Methoden wie schon in DE 3640513C2 sowie EP-B 447 585 beschrieben, angewandt werden. Diese Verfahrensweise ist in Abbildung 3 dargestellt und umfaßt im wesentlichen Anionenaustauscherchromatographie, ggf. Virenfiltration, Octansäurebehandlung, Kationenaustauscherchromatographie und übliche Konzentrations-, Filtrations- und Sterilisationsschritte.

So kann die ankonzentrierte Fraktion 2 von der hydrophoben Interaktionschromatographie z.B. mittels Diafiltration auf 0,05-0,10 Mol/l NaH₂PO₄, pH 6,5, vorzugsweise auf 0,06 Mol/l NaH₂PO₄, pH 6,5, umgepuffert werden.

Zur weiteren Aufarbeitung wird diese Lösung einer Anionenaustauschchromatographie unterworfen. Hierzu wird der Anionenaustauscher mit 0,01-0,05 Mol/l NaH₂PO₄, pH 6,5. vorzugsweise mit 0,03 Mol/l NaH₂PO₄, pH 6,5, äquilibriert und die diafiltrierte Proteinlösung mit Wasser für Injektionszwecke (WFI) verdünnt, so dass auch hier eine Salzkonzentration von 0,01-0,05 Mol/l NaH₂PO₄, pH 6,5, vorzugsweise mit 0,03 Mol/l NaH₂PO₄, pH 6,5, vorliegt.

Unter diesen Bedingungen passiert eine Immunglobulin G-Rohfraktion ungebunden die Säule, während alle anderen Proteine an die chromatographische Phase binden.

Mit einem Puffer hoher Salzkonzentration (0,02 Mol/l NaH₂PO₄, 1,0 Mol/l NaCl, pH 6,5) werden die gebundenen Proteine eluiert.

Die Immunglobulin-G Rohfraktion wird gegebenenfalls über einen Virenfilter filtriert, mittels Ultra-/Diafiltration ankonzentriert und auf 0,01-0,05 Mol/l Natriumacetat, pH 5,5, vorzugsweise 0,02 Mol/l Natriumacetat, pH 5,5, umgepuffert.

Diese eingestellte Rohfraktion besteht aus > 99 % Immunglobulin-G, ist jedoch mit proteolytischen Enzymen verunreinigt.

Diese können gemäß EP 0447585B1 mit Oktansäure entfernt werden. Hierzu wird die konditionierte Lösung mit 0,4 bis 1,5 Vol%, vorzugsweise 0,8 bis 1,0, insbesondere 0,8 % Oktansäure behandelt und unter Zusatz von CaCl₂ filtriert.
Anschließend erfolgt eine Virusinaktivierung mit 0,3 % Tri-n-butylphosphat und 1 % Tween 80. Nach Abschluß der Reaktionszeit wird die Lösung auf eine Leitfähigkeit eines 0,02 Mol/l Natriumacetat, pH 5,0,-Puffers mit Wasser für Injektionszwecke eingestellt.

Die Agentien der Oktansäurebehandlung und Virusinaktivierung sowie die in der Lösung enthaltenen IgG-Aggregate werden durch Chromatographie an einem Kationenaustauscher entfernt. Hierzu trägt man die eingestellte Immunglobulin G-Lösung auf den äquilibrierten Kationenaustauscher auf, wäscht mit 0,02 Mol/l Natriumacetat, pH 5,0, die Agentien aus und eluiert die Immunglobulin G-Wertfraktion mit einem Puffer, bestehend aus 0,02 Mol/l Natriumacetat, 0,3 Mol/l Natriumchlorid, pH 5,0. Die Reinigung der Säule erfolgt durch 0,02 Mol/l Natriumacetat, 1,0 Mol/l Natriumchlorid, pH 5,0 und 1,0 Mol/l NaOH.

Die Immunglobulin G-Fraktion wird gegen 0,3 Mol/l Glycin, pH 5,0 diafiltriert und auf eine Proteinkonzentration von 50 g/l ankonzentriert.

Analog kann das hochreine, iv-verträgliche IgG-Präparat auch nach anderen bekannten Verfahren aufgearbeitet werden.

Die so hergestellten intravenös verträglichen Immunglobulin G-Präparate weisen gegenüber herkömmlichen Lösungen, die üblicherweise durch Präzipitationen aus Plasma gewonnen werden, einige Vorteile auf. So entspricht beispielsweise die IgG-Subklassenzusammensetzung der natürlichen, im Plasma vorkommenden Verteilung. Der IgA-Gehalt ist kleiner 0,15 % und der IgM-Gehalt kleiner 0,02 % des Gesamtproteingehaltes. Der Anteil an IgG-Aggregaten ist kleiner 0,25 %. Mögliche, zu Unverträglichkeitsreaktionen führende Verunreinigungen, wie Prekallikreinaktivator, Prekallikrein, Kallikrein, Kininogen, Plasmin, Plasminogen und Faktor XI sind nicht nachweisbar. Alle anderen analytischen Parameter entsprechen der Europäischen Pharmakopoe für i. v. Immunglobulin G-Präparate.

Somit weist das so gewonnene Präparat alle erforderlichen Kriterien auf und unterscheidet sich von dem bekannten Produkt zum einen durch den höheren IgG-Anteil und ferner hinsichtlich der Subklassenzusammensetzung.

### b) Albumin, Antithrombin III, Transferrin

Aus der erfindungsgemäß hergestellten Fraktion 1 lassen sich beispielsweise Antithrombin-III (AT-III), Transferrin und Albumin gewinnen.

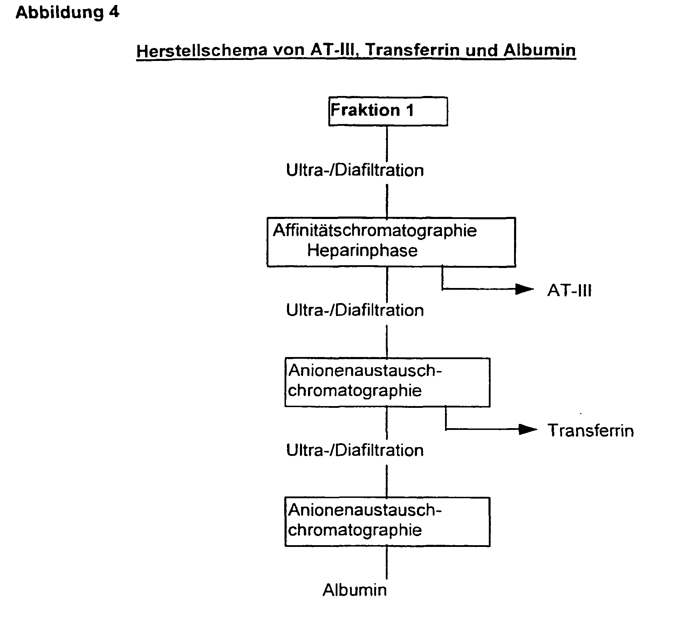

Ein entsprechendes Verfahren ist in Abbildung 4 dargestellt und umfaßt im wesentlichen Affinitätschromatographie, Anionenaustauscherchromatographie, Virusinaktivierung sowie übliche Filtrations-, Konzentrations- und Sterilisationsschritte.

So läßt sich aus der ultra- und diafiltrierten Fraktion 1 mittels Affinitätschromatographie an einem Heparinträger Antithrombin III aus diesen enthaltenden Ausgangsprodukten gewinnen. Eine solche Vorgehensweise ist in der EP-A 844 254 als Vorreinigungsstufe zur Gewinnung von von Unreinheiten befreitem Antithrobin III beschrieben, wobei dort anschließend eine Hitze- und/oder Metallchelatbehandlung erfolgt. Im erfingungsgemäßen Verfahren kann die Affinitätschromatographie somit ebenfalls durchgeführt werden, wobei hier eine niedrig konzentrierte Kochsalzlösung wie z.B. 0,1 bis 0,2 Mol/l Natriumchlorid zur Bindung von Antithrobin III und eine höhere Kochsalzkonzentration wie z.B. 1,0 bis 2,0 Mol/l Natriumchlorid zur Eluierung führen. So kann man die Fraktion 1 auf ein Puffermilieu von 0,02 Mol/l, NaH₂PO₄, 0,150 Mol/l NaCl, pH 7,0, einstellen und trägt auf die Affinitätsphase auf.

Albumin und Transferrin passieren die Säule ungebunden. Man wäscht die Säule mit 0,02 Mol/l, NaH₂PO₄, 0,4 Mol/l NaCl, pH 7,0 und eluiert anschließend das an den Heparinträger gebundene AT-III mit 0,02 Mol/l, NaH₂PO₄, 2,0 Mol/l NaCl, pH 7,0. Die gewonnene AT-III Lösung wird auf physiologische Bedingungen umgepuffert und kann nach bekannten Methoden, wie z. B. Virusfiltration und Pasteurisierung zu einem therapeutisch anwendbaren Präparat weiterverarbeitet werden.

Die Albumin und Transferrin enthaltende Durchlauffraktion der Affinitätschromatographie kann gemäß DE 3733181C1 zu Transferrin und gemäß EP 0402205B1 oder EP 0792887A1 zu Albumin aufgetrennt werden.
So kann beispielsweise nach Umpufferung der o. g. Durchlauffiltration der Affinitätschromatographie auf 0,02 Mol/l Tris, 0,030 Mol/l NaCl, pH 7,0, die Lösung auf einen Anionenaustauscher aufgetragen werden. Das Transferrin wird als Durchlauffraktion aufgefangen und mittels bekannter Methoden virusinaktiviert und weiterverarbeitet. Die vom Anionenaustauscher anschließend mit 0,02 Mol/l Tris, 1,0 Mol/l NaCl, pH 7,0, eluierte Albuminfraktion kann beispielsweise auf eine Leitfähigkeit von 1,8 mS/cm mit einem Natriumacetatpuffer, pH 6,0, eingestellt und erneut einer Anionenaustauschchromatographie unterzogen werden. Durch Umstellen des pH-Wertes auf 5,2 werden eventuell vorhandene Spuren von Transferrin entfernt. Die Elution des Albumins vom Anionenaustauscher erfolgt bei pH 4,5 und einer Leitfähigkeit von 1,8 mS/cm. Die Weiterverarbeitung zu einem therapeutisch anwendbaren Albuminpräparat erfolgt nach bekannten Methoden.

Für alle in den obengenannten Verfahren angewandten chromatographischen Schritte werden bekannte Materialien verwendet.
Als Kationenaustauscher können z.B. CM-Accell®, SP-Spherodex®, SP-Trisacryl-LS® oder Fraktogel-TSK-SP 650®, Poros HS® oder S-HyperDF® oder SOURCE-30S®, CM-HyperDF® verwendet werden und die Salzkonzentration entsprechend eingestellt werden.
Als Anionenaustauscher können z.B. QMA-Accell® DEAE-Spherosil® oder DEAE-Sepharose®, PorosHQ®, Q-HyperDF®, SOURCE 30Q® eingesetzt werden.
Weitere geeignete Materialien sind CM-Accell®, SP-Sperodex-M® oder auf der Basis synthetischer Polymere hergestellte Phasen wie SP-Trisacryl-LS® und CM-Trisacryl-LS®.
Für die Affinitätschromatographie kann z.B. Heparin-Sepharose® oder Heparin- immobilisierte synthetische Polymere wie z.B. Toyopearl AF-Heparin 650M® eingesetzt werden, wobei als Puffer ebenfalls die o.g. Zusammensetzungen gewählt werden können.

Vorzugsweise werden im erfindungsgemäßen Verfahren chromatographische Medien auf der Basis synthetischer Polymere eingesetzt, wie die unter den Handelsnamen Poros®, Source®, Macroprep®, TSK®, Toyopearl® und Hyper D® kommerziell erhältlichen.
Die genannten Phasen sind substituiert als Anionen-, Kationenaustauscher, Hydrophobe-Interaktions- und Affinitätsmatrix verfügbar. Dabei bilden tertiäre oder quaternäre Aminogruppen Anionenaustauscher, Beladung mit Sulfoalkyl oder Carboxyalkylgruppen Kationenaustauscher, Substituenten wie Alkyl oder Phenyl bzw. Heparin-Gruppen Hydrophobe Phasen bzw. Affinitätsmatrices. Die jeweiligen Puffermedien und Elutionsbedingungen hierfür sind dem Fachmann bekannt.

Aufgrund ihrer relativen Druckstabilität erlauben sie auch bei geringer Partikelgröße hohe Flußraten. Damit lassen sich die beschriebenen Prozesse in kurzer Zeit und mit hohem Durchsatz ökonomisch gestalten.
Darüberhinaus bieten diese Chromatographiephasen den Vorteil, dass sie chemisch inert gegenüber sterilisierenden Agentien und somit für die Herstellung von pharmazeutischen Produkten besonders geeignet sind.

Zur Sterilisation können Behandlungen, vorzugsweise vor dem Chromatographie-Schritt, mit β-Propiolacton, TNBP/Tween, TNBP/Na-Cholat. TNBP, gegebenenfalls in Kombination mit UV-Bestrahlung oder Virenfiltration angewendet werden. Die eingesetzten Filter wie z.B. Planova®, Virosolv®, UltriporDV50® sind bekannt.

Die auf diese Weise erfindungsgemäß hergestellten Produkte können flüssig oder lyophilisiert gelagert werden.

Der Vorteil des erfindungsgemäßen Verfahrens liegt darin, daß auf schnelle und einfache Weise die genannten Ausgangsmaterialien so gereinigt werden können, daß die erhaltenen reinen Produkte ohne wesentliche Ausbeuteverluste gewonnen werden können und ihrer natürlichen Zusammensetzung im wesentlichen entsprechen.

Die Erfindung wird durch nachstehende Beispiele erläutert.

### Beispiel 1

Als Ausgangsmaterial werden 5,4 I eines von Gerinnungsfaktoren befreiten Humanplasmas eingesetzt. Die Entfernung der Gerinnungsfaktoren erfolgt nach bekannten Methoden durch Abtrennung des Kryopräzipitates und durch Adsorption der PPSB-Faktoren an DEAE-Sephadex-A50®.

Zu diesem vorbehandelten Plasma werden pro Kilogramm 1,2 Mol Ammoniumsulfat zugegeben und 5 h bei Raumtemperatur gerührt. Danach wird mit Wasser für Injektionszwecke (WFI) auf eine Leitfähigkeit von 112 mS/cm (20°C) entsprechend der Leitfähigkeit einer 0,9 molaren Ammoniumsulfatlösung verdünnt und 6-12 h weitergerührt. Nach Zugabe von 2 % (w/w) Filterhilfsmittel (z. B. Standard Super Cell) und erneutem Rühren über 1 Stunde wird die Lösung über einen Tiefenfilter, z. B. Seitz Supra 80P, geklärt.

Eine Stahlsäule (373 ml) gefüllt mit TSK-Phenyl 5PW® wird mit 0,9 Mol/l Ammoniumsulfat, 0,01 Mol/l NaH₂PO₄, pH 7,0 äquilibiert. Bei einem Fluß von 70 ml/min wird die vorbereitete und filtrierte Plasmaproteinlösung in Portionen von 370 ml aufgetragen. Nach Ende des Auftrages wird mit 5 Säulenvolumen des Äquilibrierpuffers nachgewaschen und diese Fraktion gesammelt (Fraktion 1). Während des Sammelns der Fraktion 1 erfolgt eine gleichzeitige Ultrafiltration an einer 10 KD Membran, z. B. Omega®, Pall-Filtron, wobei das Permeat in den Vorratsbehälter des Äquilibrierpuffers zurückgeführt wird.

Danach wird mit dem 4fachen Säulenvolumen eines Elutionspuffer, bestehend aus 0,3 Mol/l Ammoniumsulfat, 0,01 Mol/l NaH₂PO₄, pH 7,0, die Fraktion 2 gewonnen.
Der Elutionspuffer wird über ein Mischventil aus dem Äquilibrierpuffer (0,9 Mol/l Ammoniumsulfat, 0,01 Mol/l NaH₂PO₄, pH 7,0) und einer 0,01 Mol/l NaH₂PO₄, pH 7,0, Lösung hergestellt. Hierzu werden die beiden Puffer im Verhältnis 1:3 online gemischt.

Durch Aufgabe von 3 Säulenvolumen 0,01 Mol/l NaH₂PO₄, pH 7,0-Puffer wird die Lipoprotein-Fraktion (Fraktion 3) von der Säule abgelöst und verworfen.

Nach einem Reinigungsschritt mit dem 3fachen Säulenvolumen einer 1 Mol/l NaOH-Lösung wird die Säule mit WFI freigewaschen und erneut äquilibriert.

Zur Aufarbeitung der gesamten eingesetzten filtrierten Proteinlösung sind 26 Zyklen erforderlich, wobei die Fraktionen 1 und 2 jeweils in einem Sammelbehälter aufgefangen werden.
Danach erfolgt eine Sterilfiltration der separatierten Fraktionen 1 und 2.

| | |
|---|---|
| Ausbeute, Immunglobulin G in Fraktion 2, | 90,3 % |
| Ausbeute, Albumin in Fraktion 1, | 100 % |

### Zusammensetzung der Fraktionen 1 und 2 in der Kapillarzonenelektrophorese

| | **Fraktion 1** | **Fraktion 2** |
|---|---|---|
| γ-Globulin (%) | 0 | 61,5 |
| β-Globulin (%) | 6,1 | 13,5 |
| α₁-Globulin (%) | 4,3 | 2,1 |
| α₂-Globulin (%) | 7,7 | 13,3 |
| Albumin (%) | 81,9 | 1,8 |
| Fibrinogen (%) | 0 | 7,6 |

### Beispiel 2

Man verfährt wie in Beispiel 1.

Die von Gerinnungsfaktoren befreite, mit Ammoniumsulfat versetzte und filtrierte Proteinlösung wird, wie in Beispiel 1 beschrieben, an einer TSK-Phenyl 5PW®Säule chromatographiert.

Nach Elution der Fraktion 1 werden mit 0,01 Mol/l NaH₂PO₄, pH 7,0, die Immunglobuline und Lipoproteine in einer Fraktion von der Säule abgelöst. Die erhaltenen Fraktionen zeigen folgende Ausbeuten:

| | | |
|---|---|---|
| Fraktion 1 | Albuminausbeute | 100 % |
| Fraktion 2 | Ausbeute Immunglobulin G | 95,5 % |

### Beispiel 3

Man verfährt wie in Beispiel 1.

Die von Gerinnungsfaktoren befreite, mit Ammoniumsulfat versetzte und filtrierte Proteinlösung wird an einer 3 l Säule (180x250 mm), gefüllt mit Toyopearl-Phenyl 650 M®, chromatographiert.

Die Vorgehensweise entspricht dem Beispiel 1.
Pro Zyklus werden 2,4 l der Ausgangslösung bei einer Flußrate von 300 ml/min prozessiert. Mit dieser Säule sind 3 Zyklen zur Aufarbeitung von 4 l Plasma notwendig.

Wie in Beispiel 1 werden 3 Fraktionen gesammelt.

Die erhaltenen Fraktionen zeigen folgende Ausbeuten:

| | | |
|---|---|---|
| Fraktion 1 | Albuminausbeute | 100 % |
| Fraktion 2 | Ausbeute Immunglobulin G | 96 % |

### Beispiel 4

Man verfährt wie in Beispiel 2.

Die von Gerinnungsfaktoren befreite, mit Ammoniumsulfat versetzte und filtrierte Proteinlösung wird an einer 2,5 l Säule, gefüllt mit Toyopearl-Phenyl 650 M®, chromatographiert.

Die Vorgehensweise entspricht dem Beispiel 2.

Pro Zyklus werden 2,4 l der Ausgangslösung bei einer Flußrate von 125 ml/min in 2 Fraktionen aufgetrennt. Hierzu werden 3 Zyklen benötigt.

Die erhaltenen Fraktionen zeigen folgende Ausbeuten:

| | | |
|---|---|---|
| Fraktion 1 | Albuminausbeute | 100 % |
| Fraktion 2 | Ausbeute Immunglobulin G | 95 % |

### Beispiel 5

Man verfährt wie in den Beispielen 1, 2, 3 oder 4. Anstelle eines polyvalenten Ausgangsplasmas wird ein vorselektiertes Humanplasma mit einem hohen Titer gegen Cytomegalievirus (anti-CMV) eingesetzt und entsprechend den Beispielen 1 oder 2 oder 3 oder 4 prozessiert.

Die daraus erhaltene Fraktion 2 zeigt folgende Ausbeuten:

| | |
|---|---|
| Ausbeute Immunglobulin G | 90,5 % |
| Ausbeute Anti-CMV-Titer | 78 % |

### Beispiel 6

Man verfährt wie in den Beispielen 1, 2, 3 oder 4. Anstelle eines polyvalenten Ausgangsplasmas wird ein vorselektiertes Humanplasma mit einem hohen Titer gegen Hepatitis-B-Virus (anti-HBs) eingesetzt und entsprechend den Beispielen 1 oder 2 oder 3 oder 4 prozessiert.

Die daraus erhaltene Fraktion 2 zeigt folgende Ausbeuten:

| | |
|---|---|
| Ausbeute Immunglobulin G | 92,0 % |
| Ausbeute Anti-HBs-Titer | 82 % |

### Beispiel 7

Man verfährt wie in den Beispielen 1, 2, 3 oder 4. Anstelle eines polyvalenten Ausgangsplasmas wird ein vorselektiertes Humanplasma mit einem hohen Titer gegen Anti-D eingesetzt und entsprechend den Beispielen 1 oder 2 oder 3 oder 4 prozessiert.

Die daraus erhaltene Fraktion 2 zeigt folgende Ausbeuten:

| | |
|---|---|
| Ausbeute Immunglobulin G | 91,2 % |
| Ausbeute Anti-D-Titer | 54 % |

### Beispiel 8

Eine gemäß Beispiel 1 hergestellte Immunglobulin G-haltige Fraktion 2 wird mittels Ultra- und Diafiltration auf 0,06 Mol/l NaH₂PO₄, pH 6,5 und eine Proteinkonzentration von ca. 50 g/l eingestellt. Danach erfolgt eine Verdünnung mit Wasser für Injektionszwecke (WFI) im Verhältnis 1:2. Nach einer Rührzeit von 1 Stunde wird klär- und sterilfiltriert.

Eine Stahlsäule (383 ml), gefüllt mit einem Anionenaustauscher, z. B. Poros HQ50®, wird mit einem Puffer, 0,03 Mol/l NaH₂PO₄, pH 6,5 äquilibiert.

500 ml der konditionierten Immunglobulin G-haltigen Lösung werden mit einem Fluß von 120 ml/min auf die Säule aufgetragen. Man wäscht mit 10 Säulenvolumen des Äquilibrierpuffers nach und fängt diese Immunglobulin G-haltige Fraktion auf.

Die restlichen gebundenen Proteine werden mit 0,02 Mol/l NaH₂PO₄, 1,0 Mol/l NaCl, pH 6,5 von der Säule abgelöst. Zur Reinigung wird mit 2 Säulenvolumen 1 Mol/l NaOH gewaschen und anschließend erneut äquilibriert.

Die Gesamtmenge von 2,5 l der Ausgangslösung wird in 5 Zyklen chromatographiert.

Die Immunglobulin G-haltige Auftrags- und Durchlauffraktion wird über einen Virusfilter, z. B. Planova 35, filtriert, mittels Ultra- und Diafiltration auf 0,02 Mol/l Natriumacetat, pH 5,5 umgepuffert und auf eine Proteinkonzentration von ca. 40 g/l eingestellt. Zu dieser Lösung fügt man 0,8 % (v/v) Oktansäure zu, rührt eine Stunde und hält den pH-Wert mit 1 Mol/l NaOH auf pH 5,5. Danach fügt man 0,05 Mol CaCl₂ zu, korrigiert den pH-Wert mit 1 Mol/l NaOH auf 5,5 und rührt erneut 2 Stunden.

Anschließend wird die Lösung mit 2 % (w/v) Filterhilfsmittel, z. B. Standard Super Cell, versetzt und über einen Klärfilter, z. B. Seitz Supra 80P filtriert.

Der Filterkuchen wird 2 mal mit jeweils 100 ml 0,02 Mol/l Natriumacetat, pH 5,5 nachgewaschen.

Die filtrierte Lösung wird mit 0,3 % TNBP (Tri-n-butylphosphat) und 1 % Tween 80 (Polysorbat 80) versetzt und mindestens 8 Stunden bei Raumtemperatur gerührt.

Nach Beendigung der Reaktionszeit wird durch Verdünnen mit WFI auf eine Leitfähigkeit eines 0,02 Mol/l Natriumacetat, pH 5,0-Puffers eingestellt und der pH-Wert mit 10 %iger Essigsäure auf pH 5,0 korrigiert.

Eine Stahlsäule (388 ml), gefüllt mit einem Anionenaustauscher, z. B. Poros HS50®, wird mit einem Puffer, 0,02 Mol/l Natriumacetat, pH 5,0 äquilibiert.

2,15 I der konditionierten Immunglobulin G-Lösung werden mit einem Fluß von 120 ml/min auf die Säule aufgetragen. Man wäscht mit dem 10fachen Säulenvolumen an Äquilibrierungspuffer, um die Reagenzien der Oktansäure- und Solvent-Detergent-Behandlung zu entfernen. Diese Waschfraktion wird verworfen.

Das gebundene Immunglobulin G wird mit 0,02 Mol/l Natriumacetat, 0,3 Mol/l NaCl, pH 5,0 von der Säule losgelöst und aufgefangen.

Die Reinigung der Säule erfolgt mit 0,02 Mol/l Natriumacetat, 1,0 Mol/l NaCl, pH 5,0 gefolgt von 1,0 Mol/l NaOH.

Danach wird die Säule erneut äquilibriert. Die Gesamtmenge von 2,3 l der Ausgangslösung wird in 2 Zyklen chromatographiert.

Mittels Ultra- und Diafiltration gegen 0,3 Mol/l Glycin, pH 5,0 wird die Immunglobulin G-Fraktion isoton eingestellt, auf einen Proteingehalt von 50 g/l ankonzentriert und sterilfiltriert.
Ausbeute Immunglobulin G: 74 %

### Analytische Daten einer 5 % Immunglobulin G-Lösung

| | |
|---|---|
| Antikomplementäre Aktivität | 0,68 CH₅₀/mg Protein |
| Immunglobulin A | 0,14 % |
| Immunglobulin M | 0,02 % |
| | |

| Subklassen: | |
|---|---|
| IgG₁ | 57,6 % |
| IgG₂ | 33,3 % |
| IgG₃ | 5,5 % |
| IgG₄ | 3,6 % |
| | |
| Cellulose-Acetat-Elektrophorese | 100 % γ-Globulin |
| | |
| Prekallikreinaktivator | neg. |
| Prekallikrein | neg. |
| Kallikrein | neg. |
| Kininogen | neg. |
| Plasmin | neg. |
| Plasminogen | neg. |
| Faktor XI | neg. |

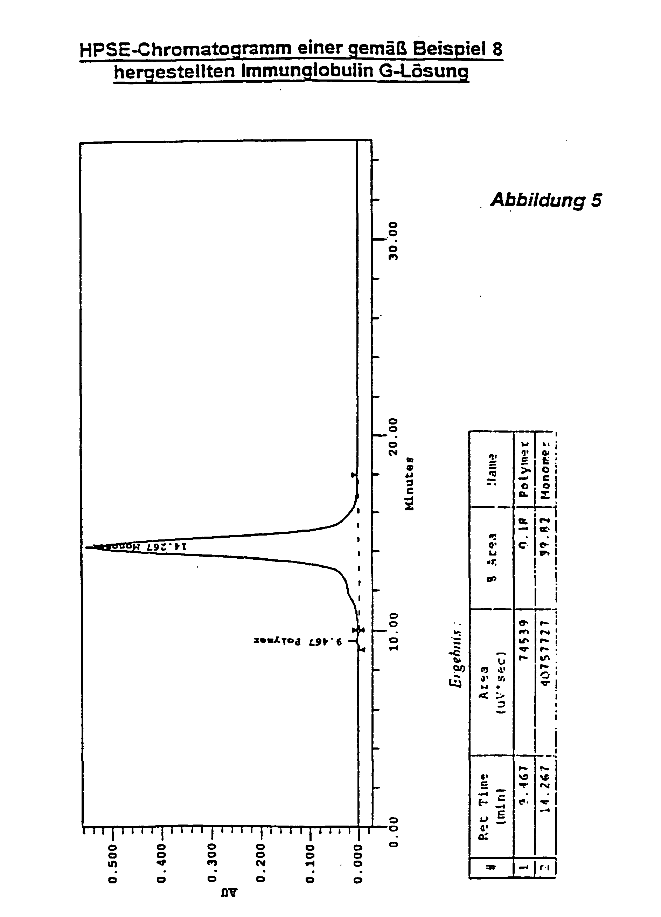

**HPSE-Chromatogramm siehe Abbildung 5**

### Beispiel 9

Eine gemäß Beispiel 1 hergestellte Albuminhaltige Fraktion 1 wird mittels Ultra- und Diafiltration auf 0,02 Mol/l, NaH₂PO₄, 0,150 Mol/l NaCl, pH 7,0 eingestellt.

Eine 50 ml Säule (1,5 x 25 cm), gefüllt mit AF-Heparin Toyopearl®, wird mit 0,02 Mol/l NaH₂PO₄, 0,150 Mol/l NaCl, pH 7,0 äquilibriert.

100 ml der konditionierten Albumin-haltigen Fraktion werden mit einem Fluß von 7 ml/min auf die Säule aufgetragen. Man spült mit 5 Säulenvolumen des Äquilibrierungspuffers nach und fängt diese Albumin-haltige Fraktion auf.

Mit 0,02 Mol/l NaH₂PO₄, 0,4 Mol/l NaCl, pH 7,0 wird die Säule nachgewaschen und diese Fraktion verworfen. Die Elution des AT-III erfolgt mit einem Puffer, bestehend aus 0,02 Mol/l NaH₂PO₄, 2,0 Mol/l NaCl, pH 7,0. Diese Wertfraktion wird mittels Ultra-/Diafiltration auf PBS umgepuffert, ankonzentriert und sterilfiltriert.

Die erhaltene AT-III Lösung zeigt folgende Daten:

| | |
|---|---|
| AT-III Ausbeute | 91 % |
| Protein | 1,1 g/l |
| AT-III-Aktivität | 805 % d.N. |
| AT-III-Antigen | 1,0 g/l |
| Albumin | < 0,006 g/l |

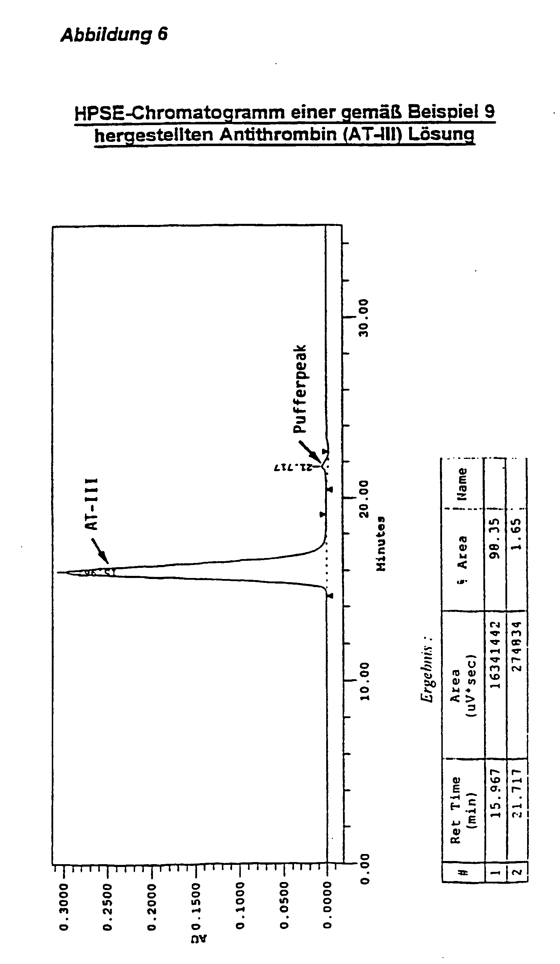

**HPSE-Chromatogramm siehe Abbildung 6**

**Tabelle 1**

| **Ermittlung der Ammoniumsulfatkonzentration zur hydrophoben Interaktionschromatographie** | | | | | |
|---|---|---|---|---|---|
| | **Protein (g/l)** | **IgG (g/l)** | **IgA (g/l)** | **IgM (g/l)** | **Albumin (g/l)** |
| Ausgang | 26,7 | 3,59 | 0,78 | 0,34 | 15,8 |
| 0,5 Mol Ammoniumsulfat | 26,7 | 3,59 | 0,78 | 0,34 | 15,8 |
| 0,7 Mol Ammoniumsulfat | 26,7 | 3,59 | 0,78 | 0,34 | 15,8 |
| 0,8 Mol Ammoniumsulfat | 26,7 | 3,59 | 0,78 | 0,34 | 15,8 |
| 1,0 Mol Ammoniumsulfat | 26,0 | 3,40 | 0,72 | 0,35 | 14,8 |
| 1,2 Mol Ammoniumsulfat | 24,9 | 1,93 | 0,38 | 0,21 | 15,7 |
| 1,4 Mol Ammoniumsulfat | 22,0 | 0,73 | 0,16 | 0,10 | 14,4 |

**Tabelle 2**

| **Beispiel für eine erfindungsgemäße Hydrophobe Interaktionschromatographie von Humanplasma bei unterschiedlichen Ammoniumsulfatkonzentrationen** | | | |
|---|---|---|---|
| | **0,7 Mol** | **0,8 Mol** | **1,0 Mol** |
| **Ausgang** Albumin (%) | 100 | 100 | 93,7* |
| IgG (%) | 100 | 100 | 94,7* |
| **Fraktion 1** Albumin (%) | > 95,0 | > 95,0 | > 95,0 |
| IgG (%) | 34,4 | < 2,6 | < 2,0 |
| **Fraktion 2** Albumin (%) | 1,4 | 1,8 | 3,3 |
| IgG (%) | 57,2 | > 95,0 | > 95,0 |

| | | | |
|---|---|---|---|
| *Präzipitation durch Ammoniumsulfat | | | |

**Tabelle 3**

| **Verteilung relevanter Plasmaproteine in Fraktionen der hydrophoben Interaktionschromatographie** | | | |
|---|---|---|---|
| | **Ausgang (%)** | **Fraktion 1 (%)** | **Fraktion 2 (%)** |
| IgG | 100 | < 5 | > 90 |
| IgA | 100 | < 2 | > 95 |
| IgM | 100 | < 5 | > 90 |
| Albumin | 100 | > 95 | < 2 |
| Transferrin | 100 | > 95 | < 2 |
| AT-III | 100 | > 90 | < 10 |
| α₁-Antritrypsin | 100 | > 90 | < 5 |
| Lipide/Lipoproteine | 100 | < 5 | > 70 |

## Patentansprüche

1. Rezyklisierungsverfahren zur präparativen Fraktionierung von Plasma oder Serum durch hydrophobe Interaktionschromatographie unter Verwendung eines stufenweisen Salzgradienten, wobei wenigstens eine Immunglobulin und eine Albumin enthaltende Fraktion erhalten wird,
**dadurch gekennzeichnet, daß**
(a) eine Serum oder Plasma enthaltende Ausgangslösung in Gegenwart einer hohen Salzkonzentration auf die hydrophobe Chromatographiephase aufgetragen,
(b) eine erste Albumin enthaltende Fraktion kontinuierlich gesammelt,
(c) die aus (b) erhaltene erste Fraktion kontinuierlich ankonzentriert,
(d) das aus (c) erhaltende Permeat mit hoher Salzkonzentration kontinuierlich dem Vorratsgefäß der Pufferlösung 1 rückgeführt, dann
(e) mit einem Mischpuffer mit niedriger Salzkonzentration, hergestellt aus der rezyklierten Pufferlösung 1 mit hoher Salzkonzentration aus (d) und einer salzfreien Pufferlösung 2, oder der Pufferlösung 2 alleine eluiert, und
(f) das Eluat als eine zweite Immunglobulin enthaltende Fraktion gesammelt
werden.

2. Rezyklisierungsverfahren nach Anspruch 1,
**dadurch gekennzeichnet, daß**
das Salz Ammoniumsulfat ist.

3. Rezyklisierungsverfahren nach Anspruch 2,
**dadurch gekennzeichnet, daß**
die in Schritt (a) verwendete Ammoniumsulfatkonzentration 0,6 bis <1,4 Mol/l und die in Schritt (e) verwendete Ammoniumsulfatkonzentration 0 bis 0,4 Mol/l beträgt.

4. Rezyklisierungsverfahren nach Anspruch 2 oder 3,
**dadurch gekennzeichnet, daß**
die in Schritt (a) verwendete Ammoniumsulfatkonzentration 0,7 bis 1 Mol/l und die in Schritt (e) verwendete Ammoniumsulfatkonzentration 0 bis 0,3 Mol/l beträgt.

5. Rezyklisierungsverfahren nach einem der Ansprüche 2 bis 4,
**dadurch gekennzeichnet, daß**
die in Schritt (a) verwendete Ammoniumsulfatkonzentration 0,8 bis 1 Mol/l und die in Schritt (c) verwendete Ammoniumsulfatkonzentration 0 bis 0,3 Mol/l beträgt.

6. Rezyklisierungsverfahren nach Anspruch 1,
**dadurch gekennzeichnet, daß**
der Elutionsschritt (e) mit dem Mischpuffer erfolgt und danach
(f) das Eluat als eine zweite Immunglobulin enthaltende Fraktion, die im Wesentlichen frei von Lipoproteinen ist, gesammelt,
(g) mit einem Mischpuffer, hergestellt aus der rezyklierten Pufferlösung 1 mit hoher Salzkonzentration aus (d) und der salzfreien Pufferlösung 2, mit einer geringerer Salzkonzentration als der in Schritt (e) verwendete Mischpuffer, oder der Pufferlösung 2 alleine eluiert, und
(h) das Eluat als eine dritte Lipoprotein enthaltende Fraktion gesammelt
werden.

7. Rezyklisierungsverfahren nach Anspruch 6,
**dadurch gekennzeichnet, daß**
das Salz Ammoniumsulfat ist.

8. Rezyklisierungsverfahren nach Anspruch 7,
**dadurch gekennzeichnet, daß**
die in Schritt (a) verwendete Ammoniumsulfatkonzentration 0,7 bis 1 Mol/l, die in Schritt (e) verwendete Ammoniumsulfatkonzentration 0,3 bis 0,1 Mol/l und die in Schritt (g) verwendete Ammoniumsulfatkonzentration <0,1 bis 0 Mol/l beträgt.

9. Rezyklisierungsverfahren nach Anspruch 7 oder 8,
**dadurch gekennzeichnet, daß**
die in Schritt (e) verwendete Ammoniumsulfatkonzentration 0,4 bis 0,1 Mol/l und die in Schritt (g) verwendete Ammoniumsulfatkonzentration 0 bis <0,1 Mol/l beträgt.

10. Rezyklisierungsverfahren nach Anspruch 7,
**dadurch gekennzeichnet, daß**
die in Schritt (a) verwendete Ammoniumsulfatkonzentration 0,9 Mol/l und die in Schritt (e) verwendete Ammoniumsulfatkonzentration 0,3 Mol/l beträgt und in Schritt (g) die Pufferlösung 2 mit einer Ammoniumsulfatkonzentration von 0 Mol/l verwendet wird.

11. Rezyklisierungsverfahren nach Anspruch 1,
**dadurch gekennzeichnet, daß**
die Ausgangslösung und die hydrophobe Chromatographiephase bei Beginn der Fraktionierung auf die hohe Salzkonzentration der Pufferlösung 1 eingestellt wird.

12. Rezyklisierungsverfahren nach einem der Ansprüche 1 bis 11,
**dadurch gekennzeichnet, daß**
als Ausgangsmaterial ein Plasma oder Serum humanen oder tierischen Ursprungs verwendet wird.

13. Rezyklisierungsverfahren nach einem der Ansprüche 1 bis 11,
**dadurch gekennzeichnet, daß**
als Ausgangsmaterial ein Plasma eingesetzt wird, aus welchem die Gerinnungsfaktoren des PPSB-Komplexes (Faktoren II, VII, IX und X) abgetrennt sind.

14. Rezyklisierungsverfahren nach einem der Ansprüche 1 bis 11,
**dadurch gekennzeichnet, daß**
als Ausgangsmaterial ein Plasma eingesetzt wird, aus welchem der Gerinnungsfaktor VIII abgetrennt ist.

15. Rezyklisierungsverfahren nach einem der Ansprüche 1 bis 11,
**dadurch gekennzeichnet, daß**
als Ausgangsmaterial polyvalentes humanes Plasma verwendet wird.

16. Rezyklisierungsverfahren nach einem der Ansprüche 1 bis 11,
**dadurch gekennzeichnet, daß**
als Ausgangsmaterial ein selektiertes humanes Plasma mit hohen Antikörpertitern gegen virale, bakterielle oder zelluläre Antigene verwendet wird.

17. Rezyklisierungsverfahren nach einem der Ansprüche 1 bis 16,
**dadurch gekennzeichnet, daß**
als hydrophobe Chromatographiephase Phenyl- oder Alkyl-substituierte Copolymere aus Glycidylmethacrylat und Ethylenglycoldimethacrylat, Copolymere aus Polystyrol und Divinylbenzol oder mit Dextran oder Polymeren beschichtete Kieselgele verwendet werden.

18. Rezyklisierungsverfahren nach einem der Ansprüche 1 bis 17,
**dadurch gekennzeichnet, daß**
als hydrophobe Chromatographiephase Phenyl-substituierte Copolymere aus Glycidylmethacrylat und Ethylenglycoldimethacrylat verwendet werden.

19. Rezyklisierungsverfahren nach einem der Ansprüche 1 bis 18,
**dadurch gekennzeichnet, daß**
die hydrophobe Chromatographiephase nach jeweils einem Zyklus mit Natronlauge aus einem Vorratsgefäß 3 behandelt wird.

20. Rezyklisierungsverfahren nach einem der Ansprüche 1 bis 19,
**dadurch gekennzeichnet, daß**
die erhaltene erste Fraktion zu therapeutisch anwendbarem, hochreinem Antithrombin III, Transferrin und/oder Albumin weiterverarbeitet wird.

21. Rezyklisierungsverfahren nach einem der Ansprüche 1 bis 19,
**dadurch gekennzeichnet, daß**
die erhaltene zweite Fraktion zu therapeutisch anwendbarem, hochreinem IgG weiterverarbeitet wird.

## Claims

1. Recycling process for the preparative fractionation of plasma or serum by hydrophobic interaction chromatography using a stepwise salt gradient, in which at least one fraction containing an immunoglobulin and an albumin is obtained,
**characterised in that**
(a) a starting solution containing serum or plasma is applied in the presence of a high salt concentration to the hydrophobic chromatography phase,
(b) a first, albumin-containing fraction is continuously collected,
(c) the first fraction obtained from (b) is continuously concentrated,
(d) the permeate with a high salt concentration obtained from (c) is continuously recycled to the storage vessel of the buffer solution 1, following which
(e) it is eluted with a mixed buffer of low salt concentration prepared from the recycled buffer solution 1 with high salt concentration from (d) and a salt-free buffer solution 2, or from the buffer solution 2 alone, and
(f) the eluate is collected as a second, immunoglobulin-containing fraction.

2. Recycling process according to claim 1, **characterised in that** the salt is ammonium sulfate.

3. Recycling process according to claim 2, **characterised in that** the ammonium sulfate concentration used in step (a) is 0.6 to <1.4 mole/l and the ammonium sulfate concentration used in step (e) is 0 to 0.4 mole/l.

4. Recycling process according to claim 2 or 3, **characterised in that** the ammonium sulfate concentration used in step (a) is 0.7 to 1 mole/l and the ammonium sulfate concentration used in step (e) is 0 to 0.3 mole/l.

5. Recycling process according to one of claims 2 to 4, **characterised in that** the ammonium sulfate concentration used in step (a) is 0.8 to 1 mole/l and the ammonium sulfate concentration used in step (c) is 0 to 0.3 mole/l.

6. Recycling process according to claim 1, **characterised in that** the elution step (e) is carried out with the mixed buffer, following which
(f) the eluate is collected as a second, immunoglobulin-containing fraction that is substantially free of lipoproteins,
(g) is eluted with a mixed buffer prepared from the recycled buffer solution 1 of high salt concentration from (d) and the salt-free buffer solution 2 with a lower salt concentration than the mixed buffer used in step (e), or from the buffer solution 2 alone, and
(h) the eluate is collected as a third, lipoprotein-containing fraction.

7. Recycling process according to claim 6, **characterised in that** the salt is ammonium sulfate.

8. Recycling process according to claim 7, **characterised in that** the ammonium sulfate concentration used in step (a) is 0.7 to 1 mole/l, the ammonium sulfate concentration used in step (e) is 0.3 to 0.1 mole/l and the ammonium sulfate concentration used in step (g) is <0.1 to 0 mole/l.

9. Recycling process according to claim 7 or 8, **characterised in that** the ammonium sulfate concentration used in step (e) is 0.4 to 0.1 mole/l and the ammonium sulfate concentration used in step (g) is 0 to <0.1 mole/l.

10. Recycling process according to claim 7, **characterised in that** the ammonium sulfate concentration used in step (a) is 0.9 mole/l and the ammonium sulfate concentration used in step (e) is 0.3 mole/l, and in step (g) the buffer solution 2 with an ammonium sulfate concentration of 0 mole/l is used.

11. Recycling process according to claim 1, **characterised in that** the starting solution and the hydrophobic chromatography phase are adjusted at the start of the fractionation to the high salt concentration of buffer solution 1.

12. Recycling process according to one of claims 1 to 11, **characterised in that** a plasma or serum of human or animal origin is used as starting material.

13. Recycling process according to one of claims 1 to 11, **characterised in that** a plasma from which the coagulating factors of the PPSB complex (factors II, VII, IX and X) have been separated is used as starting material.

14. Recycling process according to one of claims 1 to 11, **characterised in that** a plasma from which the coagulating factor VIII has been separated is used as starting material.

15. Recycling process according to one of claims 1 to 11, **characterised in that** polyvalent human plasma is used as starting material.

16. Recycling process according to one of claims 1 to 11, **characterised in that** a selected human plasma with high antibody titres against viral, bacterial or cellular antigens is used as starting material.

17. Recycling process according to one of claims 1 to 16, **characterised in that** phenyl-substituted or alkyl-substituted copolymers of glycidyl methacrylate and ethylene glycol dimethacrylate, copolymers of polystyrene and divinylbenzene, or silica gels coated with dextran or polymers, are used as the hydrophobic chromatography phase.

18. Recycling process according to one of claims 1 to 17, **characterised in that** phenyl-substituted copolymers of glycidyl methacrylate and ethylene glycol dimethacrylate are used as the hydrophobic chromatography phase.

19. Recycling process according to one of claims 1 to 18, **characterised in that** the hydrophobic chromatography phase is treated after each cycle with sodium hydroxide from a storage vessel 3.

20. Recycling process according to one of claims 1 to 19, **characterised in that** the obtained first fraction is worked up into therapeutically useable, very pure antithrombin III, transferrin and/or albumin.

21. Recycling process according to one of claims 1 to 19, **characterised in that** the obtained second fraction is worked up into therapeutically useable, very pure IgG.

## Revendications

1. Procédé de recyclisation pour le fractionnement préparatoire de plasma ou de sérum par chromatographie interactive hydrophobe avec utilisation d'un gradient de sel progressif, permettant d'obtenir au moins une fraction contenant une immunoglobuline et une albumine,
**caractérisé en ce que**,
(a) une solution initiale contenant du sérum ou du plasma est chargée dans une phase chromatographique hydrophobe en présence d'une forte concentration de sel,
(b) une fraction contenant une première albumine est collectée de façon continue,
(c) la première fraction obtenue à partir de (b) est concentrée de façon continue,
(d) le perméat obtenu à partir de (c) est réacheminé en continu à une forte concentration en sel jusqu'au récipient de stockage de la solution tampon, puis,
(e) élué avec un tampon de mélange à faible concentration de sel, fabriqué à partir d'une solution tampon recyclisée 1 à forte concentration en sel issue de (d) et d'une solution tampon 2 sans sel, ou d'une solution tampon 2, et
(f) l'éluat est collecté sous la forme d'une fraction contenant une deuxième immunoglobuline.

2. Procédé de recyclisation selon la revendication 1, **caractérisé en ce que** le sel est un sulfate d'ammonium.

3. Procédé de recyclisation selon l'une des revendications 1 ou 2, **caractérisé en ce que** la concentration de sulfate d'ammonium utilisée à l'étape (a) va de 0, 6 à <1,4 mole/l et que la concentration de sulfate d'ammonium utilisée à l'étape (e) va de 0 à 0,4 mole/l.

4. Procédé de recyclisation selon l'une des revendications 2 ou 3, **caractérisé en ce que** la concentration de sulfate d'ammonium utilisée à l'étape (a) va de 0,7 à 1 mole/l et que la concentration de sulfate d'ammonium utilisée à l'étape (e) va de 0 à 0,3 mole/l.

5. Procédé de recyclisation selon l'une des revendications 2 à 4, **caractérisé en ce que** la concentration de sulfate d'ammonium utilisée à l'étape (a) va de 0,8 à 1 mole/l et que la concentration de sulfate d'ammonium utilisée à l'étape (c) va de 0 à 0,3 mole/l.

6. Procédé de recyclisation selon la revendication 1, **caractérisé en ce que** l'étape d'élution (e) se fait avec un tampon de mélange puis
(f) l'éluat est collecté sous la forme d'une fraction contenant une deuxième immunoglobuline, qui est pour l'essentiel vierge de lipoprotéines,
(g) élué avec un tampon de mélange, fabriqué à partir de la solution tampon recyclisée 1 avec une forte concentration de sel issue de (d) et de la solution tampon 2 sans sel, à une concentration de sel plus faible que le tampon de mélange utilisé à l'étape (e), ou la solution tampon 2 seule, et
(h) l'éluat est collecté sous la forme d'une fraction contenant une troisième lipoprotéine.

7. Procédé de recyclisation selon la revendication 6, **caractérisé en ce que** le sel est du sulfate d'ammonium.

8. Procédé de recyclisation selon la revendication 7, **caractérisé en ce que** la concentration de sulfate d'ammonium utilisée à l'étape (a) va de 0,7 à 1 mole/l, que la concentration de sulfate d'ammonium utilisée à l'étape (e) va de 0,3 à 0,1 mole/l et que la concentration de sulfate d'ammonium utilisée à l'étape (g) va de <0,1 à 0 mole/l.

9. Procédé de recyclisation selon l'une des revendications 7 ou 8, **caractérisé en ce que** la concentration de sulfate d'ammonium utilisée à l'étape (e) va de 0,4 à 0,1 mole/l et que la concentration de sulfate d'ammonium utilisée à l'étape (g) va de 0 à <0,1 mole/l.

10. Procédé de recyclisation selon la revendication 7, **caractérisé en ce que** la concentration de sulfate d'ammonium utilisée à l'étape (a) est de 0,9 mole/l, que la concentration de sulfate d'ammonium utilisée à l'étape (e) est de 0,3 mole/l et que la solution tampon 2 est utilisée à l'étape (g) selon une concentration de sulfate d'ammonium de 0 mole/l.

11. Procédé de recyclisation selon la revendication 1, **caractérisé en ce que** la solution initiale et la phase chromatographique hydrophobe sont ajustées au début du fractionnement à hauteur de la concentration de sel de la solution tampon 1.

12. Procédé de recyclisation selon l'une des revendications 1 à 11, **caractérisé en ce qu'**on utilise comme matériau de départ un plasma ou un sérum d'origine humaine ou animale.

13. Procédé de recyclisation selon l'une des revendications 1 à 11, **caractérisé en ce qu'**on utilise du plasma comme matériau de départ, duquel les facteurs de coagulation du complexe PPSB (facteurs II, VII, IX et X) ont été séparés.

14. Procédé de recyclisation selon l'une des revendications 1 à 11, **caractérisé en ce qu'**on utilise comme matériau de départ du plasma duquel le facteur de coagulation VIII a été séparé.

15. Procédé de recyclisation selon l'une des revendications 1 à 11, **caractérisé en ce qu'**on utilise comme matériau de départ du plasma humain polyvalent.

16. Procédé de recyclisation selon l'une des revendications 1 à 11, **caractérisé en ce qu'**on utilise comme matériau de départ du plasma humain sélectionné avec un titrage d'anticorps élevé contre des antigènes viraux, bactériens ou cellulaires.

17. Procédé de recyclisation selon l'une des revendications 1 à 16, **caractérisé en ce qu'**on utilise comme phase chromatographique hydrophobe des copolymères de méthacrylate de glycidyle et de diméthacrylate d'éthylène glycol avec substitution de phényle ou d'alkyle, des copolymères de polystyrol et de divinylbenzol ou avec du dextrane ou des gels de silice enrobés de polymères.

18. Procédé de recyclisation selon l'une des revendications 1 à 17, **caractérisé en ce qu'**on utilise comme phase chromatographique hydrophobe des copolymères de méthacrylate de glycidyle et de méthacrylate d'éthylène glycol avec substitution de phényle.

19. Procédé de recyclisation selon l'une des revendications 1 à 18, **caractérisé en ce que** la phase chromatographique hydrophobe est traitée après chaque cycle avec de la soude caustique provenant du récipient de stockage 3.

20. Procédé de recyclisation selon l'une des revendications 1 à 19, **caractérisé en ce que** la première fraction obtenue est transformée ensuite en antithrombine II, transferrine et/ou albumine hautement purifiée applicable au plan thérapeutique.

21. Procédé de recyclisation selon l'une des revendications 1 à 19, **caractérisé en ce que** la deuxième fraction obtenue est transformée en IgG hautement purifiée applicable au plan thérapeutique.
